# EUROPEAN PATENT APPLICATION

(11) **EP 4 781 959 A1**
(43) Date of publication of application: **29.07.2026**
(21) Application number: 24888510.5
(22) Date of filing: 21.10.2024
(51) Int. Cl.: A61F 13/15, A61F 13/532

(54) **ABSORBENT-ARTICLE MANUFACTURING DEVICE**

(30) Priority: 09.11.2023 JP 2023191487
(71) Applicant: Zuiko Corporation, Ibaraki-shi Osaka 5670082 (JP)
(72) Inventor: IWAMURA, Yosuke, Ibaraki-shi, Osaka 567-0082 (JP)
(74) Representative: SSM Sandmair
(86) International application number: PCT/JP2024/037418
(87) International publication number: WO 2025/100212

(57) **Abstract**

There is provided an apparatus for manufacturing absorbent body that can suppress scattering of an absorbent material and oozing of an adhesive.

Recess parts 13a to 13c are formed on an outer circumferential surface 14k of a first drum 14 that conveys a first sheet 2, and suction holes 13x are formed on the bottom surfaces of the recess parts 13a to 13c. Spray ports 12a to 12c of a granular material supply device 12 are arranged so as to face the recess parts 13a to 13c, and a guide roll 18 that guides a second sheet 4 is disposed closer to a downstream side in a rotation direction of the first drum 14 than the spray ports 12a to 12c such that one principal surface of the second sheet 4 overlaps one principal surface of the first sheet 2 to form a sheet laminated body 6. Before the one principal surface of the second sheet 4 overlaps the one principal surface of the first sheet 2, adhesive application devices 19a and 19b apply adhesives to the one principal surface of the first sheet 2 and/or the one principal surface of the second sheet 4. A press unit 31 compresses the sheet laminated body 6 in a thickness direction.

## Description

### Technical Field

The present invention relates to an apparatus for manufacturing absorbent body, and, more particularly, to a technique of manufacturing an absorber used for absorbent goods.

### Background Art

Absorbers are used for absorbent goods such as disposable diapers, disposable pants, sanitary napkins, and incontinence pads. In the absorber, an absorbent material that absorbs and holds liquid is sandwiched between two sheets.

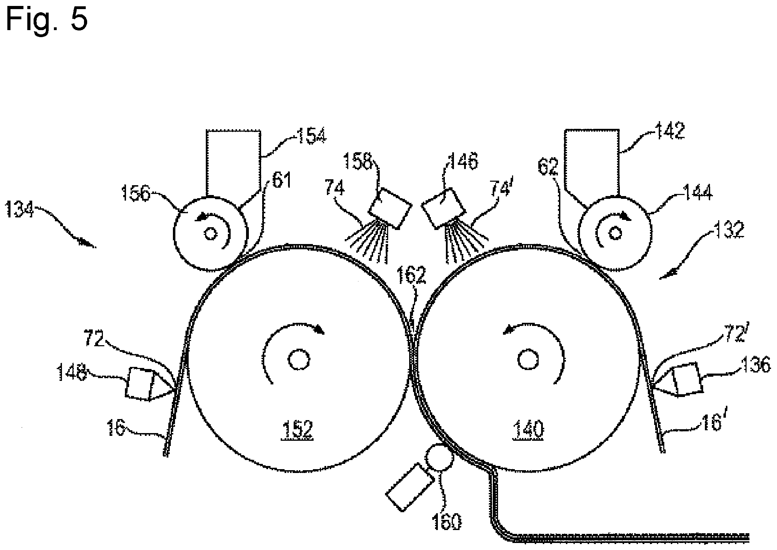

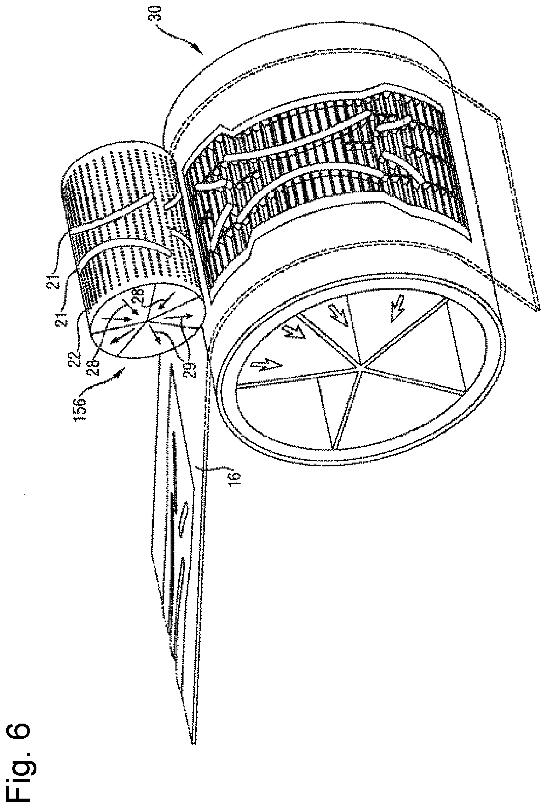

For example, Fig. 5 illustrates a schematic view of an apparatus for manufacturing absorbent body that manufactures an absorber used for absorbent goods. Fig. 6 illustrates a partial perspective view of the apparatus for manufacturing absorbent body. As illustrated in Figs. 5 and 6, in the apparatus for manufacturing absorbent body, a first printing unit 134 deposits an absorbent material on a first sheet 16, a second printing unit 132 deposits an absorbent material on a second sheet 16', and the first sheet 16 and the second sheet 16' are bonded to each other to form an absorber.

When an auxiliary adhesive application device 148 applies a thermoplastic adhesive material 72 to the first sheet, a movable surface 30 of a first drum 152 conveys the first sheet 16 while suctioning the first sheet 16, and the first sheet passes between the first drum 152 and a first print roll 156, the first printing unit 134 deposits the absorbent material on the first sheet 16, and an adhesive application device 158 applies an adhesive 74 thereon.

Holes 22 are formed in the outer circumferential surface of the first print roll 156, and a plurality of chambers communicating with the holes 22 are formed inside the first print roll 156. When the holes 22 in the outer circumferential surface of the first print roll 156 face a hopper 154 that stores the absorbent material, the holes 22 communicate with a chamber of a negative pressure as indicated by an arrow 28 to suction and hold the absorbent material. When the holes 22 in the outer circumferential surface of the first print roll 156 face the first sheet 16 suctioned and conveyed by the movable surface 30 of the first drum 152, the holes 22 communicate with a chamber to which a positive pressure is applied as indicated by an arrow 29, and an absorbent material 61 held in the holes 22 moves to and is deposited on the first sheet 16.

The second printing unit 132 is configured similarly to the first printing unit 134, and an auxiliary adhesive application device 136 applies a thermoplastic adhesive material 72' to the second sheet 16', an absorbent material 62 moves to and is deposited on the second sheet 16' via a hopper 142 and a print roll 144, and an adhesive application device 146 applies an adhesive 74' thereon.

The first sheet 16 and the second sheet 16' on which the absorbent material has been deposited are bonded to each other at a nip 162, then are conveyed along a second drum 140, and, when passing between an auxiliary bonding roll 160 and the second drum 140, press areas of the first sheet 16 and the second sheet 16' that do not contain the absorbent material (see, for example, Patent Literature 1).

### Citation List

### Patent Literature

Patent Literature 1: JP 2017-507705 W

### Summary of Invention

### Technical Problem

When a conveyance speed of the sheets 16 and 16' is increased to increase production efficiency, a time taken by the absorbent materials 61 and 62 to move from the print rolls 156 and 144 to the sheets 16 and 16' is shortened, and therefore the apparatus for manufacturing absorbent body needs to increase the positive pressure to be applied to the holes 22 of the print rolls 156 and 144 at a time of movement of the absorbent materials 61 and 62. In this case, the force to push out the absorbent materials 61 and 62 from the holes 22 increases, and therefore there is a problem that part of the pushed-out absorbent materials 61 and 62 scatters around.

Furthermore, pressing at a time when the first sheet 16 and the second sheet 16' pass between the auxiliary bonding roll 160 and the second drum 140 oozes out from the sheets 16 and 16' the adhesive disposed between the first sheet 16 and the second sheet 16'. When the adhesive oozes out, failures such as adhesion and winding of the sheets 16 and 16' to and around a roll occur, and therefore it is necessary to periodically stop and clean a manufacturing line, which leads to a decrease in production efficiency.

In view of such circumstances, an object to be achieved by the present invention is to provide an apparatus for manufacturing absorbent body that can suppress scattering of an absorbent material and oozing of an adhesive.

### Solution to Problem

To solve the above problem, the present invention provides an apparatus for manufacturing absorbent body configured as follows.

An apparatus for manufacturing absorbent body includes: (a) a first drum that includes an outer circumferential surface on which a suction hole has been formed, and conveys a first sheet wound around the outer circumferential surface while suctioning the first sheet; (b) a granular material supply device that includes a spray port arranged so as to face the outer circumferential surface of the first drum, and sprays a granular material containing an absorbent material from the spray port to one principal surface of the first sheet; (c) a guide roll that is disposed closer to a downstream side in a rotation direction of the first drum than the spray port of the granular material supply device so as to face the outer circumferential surface of the first drum, and guides a second sheet such that one principal surface of the second sheet overlaps the one principal surface of the first sheet on which the granular material has been sprayed to form a sheet laminated body; (d) an adhesive application device that applies an adhesive to one or both of the one principal surface of the first sheet and the one principal surface of the second sheet before the one principal surface of the second sheet overlaps the one principal surface of the first sheet; and (e) a press unit that compresses the sheet laminated body in a thickness direction. In a region of the outer circumferential surface of the first drum around which the first sheet is wound, a recess part that is continuous in a circumferential direction of the first drum is formed, the suction hole is formed in a bottom surface of the recess part, and the spray port of the granular material supply device is disposed so as to face the recess part.

According to the above configuration, a portion of the first sheet facing the recess part is suctioned and dented. The granular material is sprayed in this dent, so that scattering of the granular material to an outer side of a spraying region is suppressed. Furthermore, even when a spray amount of a granular material per unit area increases, the granular material tends to stay in the dent of the first sheet, and thus scattering of the granular material is suppressed.

Furthermore, by forming the recess part on the outer circumferential surface of the first drum, it is possible to reduce a compression amount of portions of the first sheet and the second sheet that sandwich the granular material when the first sheet and the second sheet pass between a guide roll and the first drum. Consequently, it is possible to prevent the adhesive from oozing out even when the amount of the adhesive to be applied to the portions sandwiching the granular material is increased, and it is easy to strengthen fixation of the granular material.

Preferably, a depth to the bottom surface of the recess part of the first drum is smaller than a thickness of the first sheet.

In this case, a dent at a portion of the first sheet facing the recess part is shallow, so that it is possible to more precisely spray the granular material to a predetermined position of the first sheet.

Preferably, the apparatus for manufacturing absorbent body further includes (f) a heater that is inside the press unit or closer to an upstream side in the conveyance direction of the sheet laminated body than the press unit. The press unit compresses the sheet laminated body in the thickness direction over an entire width direction intersecting a conveyance direction of the sheet laminated body.

In this case, it is possible to heat the sheet laminated body by the heater and enhance fluidity of the adhesive, promote diffusion of the adhesive in the entire sheet laminated body, and form the absorber in which the granular material has been firmly fixed. Consequently, the granular material does not move in the absorber, so that it is possible to suppress variations in quality of the absorber.

Preferably, a plurality of the recess parts are formed at intervals from each other in the region of the outer circumferential surface of the first drum. The granular material supply device includes a plurality of the spray ports arranged so as to face the respective recess parts.

In this case, an absorber including a channel region to which the absorbent material is not sprayed can be formed between regions to which the absorbent material has been sprayed.

### Advantageous Effects of Invention

According to the present invention, it is possible to suppress scattering of an absorbent material and oozing of an adhesive.

### Brief Description of Drawings

Fig. 1 illustrates a schematic view of an apparatus for manufacturing absorbent body. (first embodiment)
Fig. 2(a) illustrates a developed view of main parts of an outer circumferential surface of a first drum, and Fig. 2(b) illustrates a schematic cross-sectional view of the main parts taken along line B-B in Fig. 2(a). (first embodiment)
Fig. 3(a) illustrates a plan view of a plate-like member, and Fig. 3(b) illustrates a schematic cross-sectional view of a welded part. (first embodiment)
Fig. 4(a) illustrates an explanatory view for a time of spraying a granular material, and Fig. 4(b) illustrates an explanatory view for a time of pressing a sheet laminated body. (first embodiment)
Fig. 5 illustrates a schematic view of an apparatus for manufacturing absorbent body. (conventional example 1)
Fig. 6 illustrates a partial perspective view of the apparatus for manufacturing absorbent body. (conventional example 1)

### Description of Embodiment

Hereinafter, an embodiment of the present invention will be described with reference to the drawings.

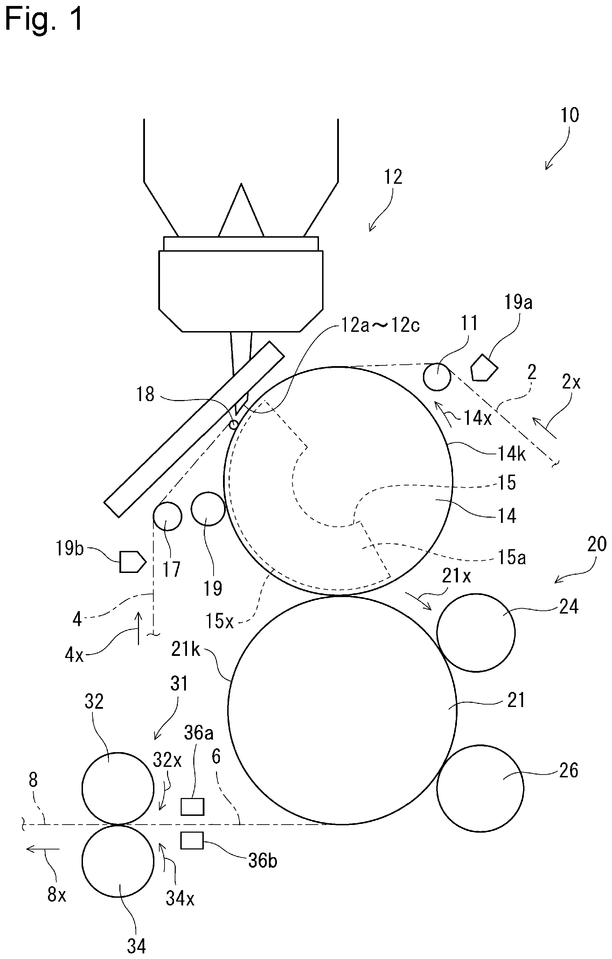

<First Embodiment> An apparatus for manufacturing absorbent body 10 according to the first embodiment will be described with reference to Figs. 1 to 4.

Fig. 1 illustrates a schematic view illustrating a configuration of the apparatus for manufacturing absorbent body 10. As illustrated in Fig. 1, in the apparatus for manufacturing absorbent body 10, a first adhesive application device 19a, a first intermediate roll 11, a first drum 14, a second drum 21, and a first press unit 31 are disposed along a conveyance path of a first sheet 2 to be conveyed in a direction indicated by an arrow 2x. A granular material supply device 12, a guide roll 18, and a pressing roll 19 are disposed around the first drum 14. Press rolls 24 and 26 are disposed around the second drum 21. Furthermore, a second adhesive application device 19b and a second intermediate roll 17 are disposed along a conveyance path of the second sheet 4 to be conveyed toward the guide roll 18 in a direction indicated by an arrow 4x.

The apparatus for manufacturing absorbent body 10 sprays a granular material to the first sheet 2 to be conveyed along an outer circumferential surface 14k of the first drum 14, overlaps the second sheet 4 thereon to form a sheet laminated body 6, and compresses the sheet laminated body 6 in a thickness direction by the first press unit 31 to form an absorber 8. As indicated by an arrow 8x, the absorber 8 is conveyed toward an unillustrated subsequent process, cut at an appropriate position, and used for absorbent goods in a singulated state.

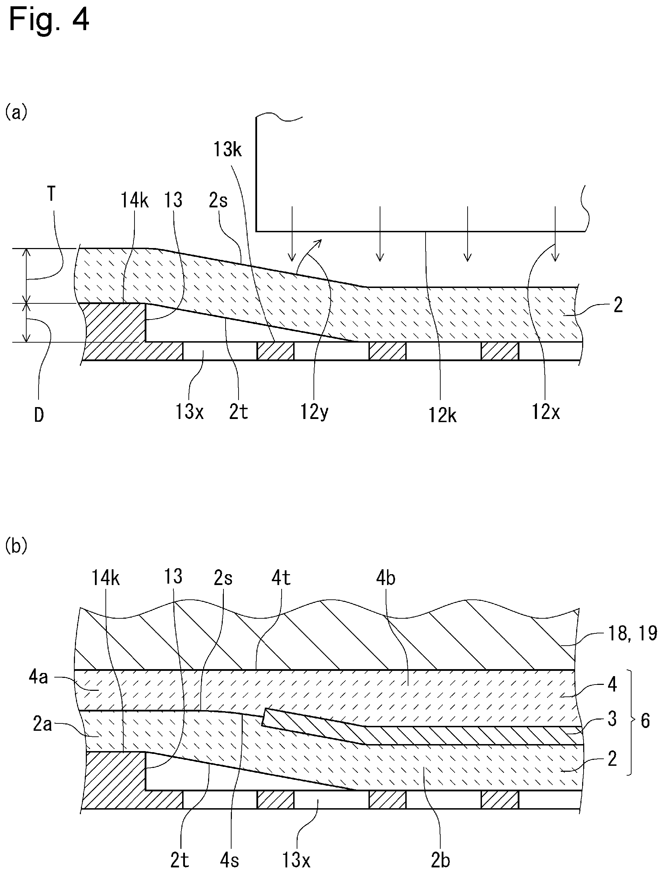

The first adhesive application device 19a is disposed so as to face one principal surface 2s (see Fig. 4) of the first sheet 2, and applies an adhesive to the one principal surface 2s of the first sheet 2 by spraying or the like.

The second adhesive application device 19b is disposed so as to face one principal surface 4s (see Fig. 4) of the second sheet 4, and applies the adhesive to the one principal surface 4s of the second sheet 4 by spraying or the like.

The outer circumferential surface 14k of the first drum 14 rotates in a direction indicated by an arrow 14x. As will be described in detail later, the first drum 14 conveys the first sheet 2 wound around the outer circumferential surface 14k while suctioning the first sheet 2.

The granular material supply device 12 includes three spray ports 12a to 12c arranged so as to face the outer circumferential surface 14k of the first drum 14, and sprays the granular material containing an absorbent material such as Super Absorbent Polymers (SAPs) from the spray ports 12a to 12c to the one principal surface 2s of the first sheet 2 conveyed along the first drum, that is, the one principal surface 2s on an opposite side to another principal surface 2t (see Fig. 4) supported by the outer circumferential surface 14k of the first drum 14. The number of the spray ports 12a to 12c may be one, two, or four or more.

The guide roll 18 is disposed closer to a downstream side in a rotation direction of the first drum 14 than the spray ports 12a to 12c of the granular material supply device 12 so as to face the outer circumferential surface 14k of the first drum 14, and guides the second sheet 4 such that the one principal surface 4s of the second sheet 4 overlaps the one principal surface 2s of the first sheet 2 on which granular material has been sprayed to form the sheet laminated body 6. The sheet laminated body 6 is formed by bonding the one principal surface 2s of the first sheet 2 and the one principal surface 2s of the second sheet 4 to each other with the adhesive applied by the first and second adhesive application devices 19a and 19b and interposed therebetween, and sandwiching a granular material 3 (see Fig. 4) between the first sheet 2 and the second sheet 4.

The granular material sprayed from the spray ports 12a to 12c of the granular material supply device 12 may scatter or move before the second sheet is overlaid on the granular material. Hence, the spray ports 12a to 12c of the granular material supply device 12 and the guide roll 18 are preferably disposed as close as possible.

The pressing roll 19 is disposed closer to the downstream side in the rotation direction of the first drum 14 than the guide roll 18 so as to face the outer circumferential surface 14k of the first drum 14, and presses the sheet laminated body 6 toward the first drum 14. Thus, the one principal surface 2s of the first sheet 2 and the one principal surface 4s of the second sheet 4 of the sheet laminated body 6 are more reliably bonded. Note that the pressing roll 19 may be provided as needed, and the apparatus for manufacturing absorbent body 10 may also employ a configuration that does not include the pressing roll 19.

The second drum 21 is disposed closer to the downstream side in the rotation direction of the first drum 14 than the pressing roll 19 so as to face the outer circumferential surface 14k of the first drum 14, and an outer circumferential surface 21k rotates in a direction indicated by an arrow 21x. The sheet laminated body 6 is wound around the outer circumferential surface 21k of the second drum 21, and conveyed from the outer circumferential surface 14k of the first drum 14.

The press rolls 24 and 26 disposed around the second drum 21 press the sheet laminated body 6 toward the second drum 21 when the sheet laminated body 6 passes between the second drum 21 and the press rolls 24 and 26. The second drum 21 and the press rolls 24 and 26 are a second press unit 20. Note that the number of the press rolls 24 and 26 may be one or three or more.

The second press unit 20 may be provided as needed, and the apparatus for manufacturing absorbent body 10 may also employ a configuration that does not include the second press unit 20.

The first press unit 31 includes a pair of press rolls 32 and 34 that rotate in synchronization with directions indicated by arrows 32x and 34x, and compresses the sheet laminated body 6 in the thickness direction when the sheet laminated body 6 having passed through the second drum 21 passes between the pair of press rolls 32 and 34.

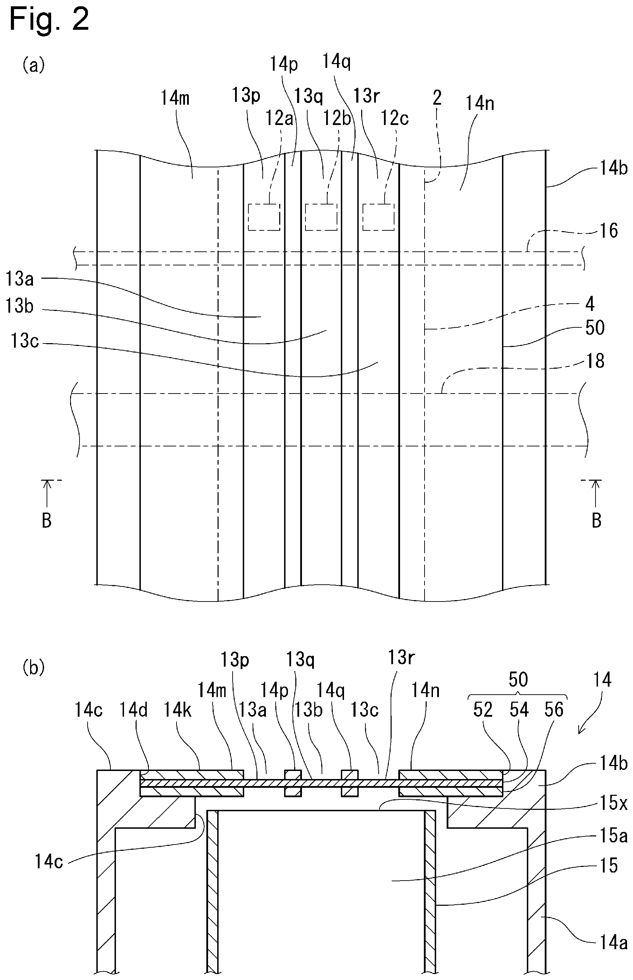

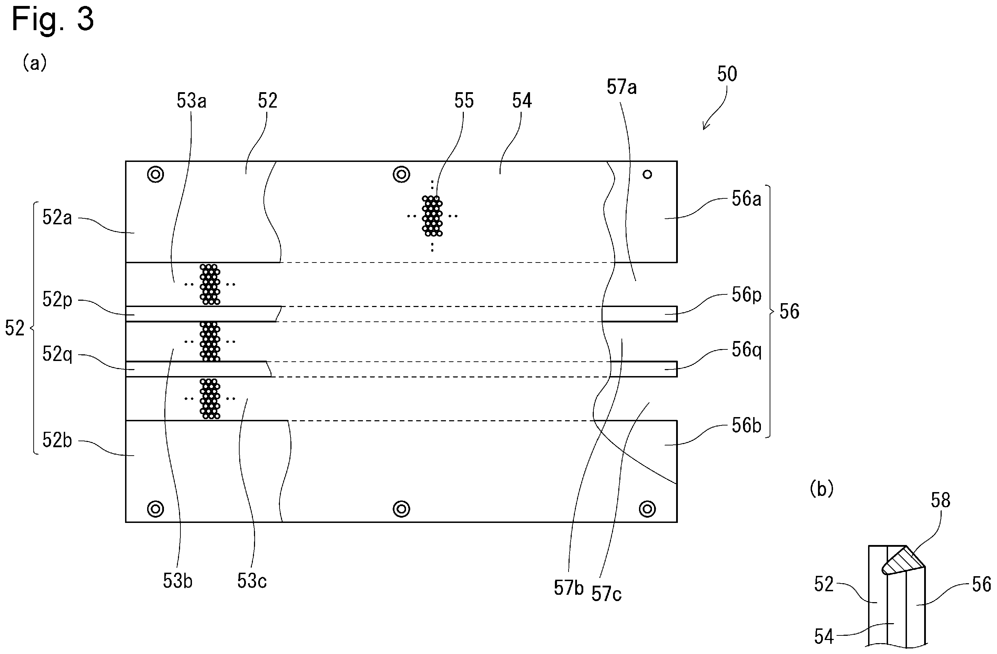

Next, the first drum 14 will be further described. Fig. 2(a) illustrates a developed view of main parts of the outer circumferential surface 14k of the first drum 14. Fig. 2(b) illustrates a schematic cross-sectional view of the main parts taken along line B-B in Fig. 2(a). Fig. 3(a) illustrates a plan view of a plate-like member 50, and Fig. 3(b) illustrates a schematic cross-sectional view of a welded part.

As illustrated in Figs. 2 and 3, the outer circumferential surface 14k of the first drum 14 is formed with the plate-like member 50, and the plate-like member 50 is configured to support the first sheet 2.

As illustrated in Fig. 2, an opening part 14c penetrating in a radial direction of the first drum 14 and extending in a circumferential direction is formed at an outer circumferential part 14b of the drum body 14a of the first drum 14, and a stepped part 14d is formed adjacent to the opening part 14c. The plate-like member 50 is fitted into the stepped part 14d so as to cover the opening part 14c, and is fixed by an unillustrated bolt or the like.

The plate-like member 50 is formed by laminating a first layer 52, a second layer 54, and a third layer 56. Groove parts 53a to 53c and 57a to 57c are formed in the first layer 52 and the third layer 56 such that the second layer 54 is exposed, and through-holes 55 communicating with the first layer 52 side and the third layer 56 side are formed in the second layer 54.

For example, portions 52a, 52b, 52p, and 52q; 56a, 56b, 56p, and 56q adjacent to the groove parts 53a to 53c and 57a to 57c of the first layer 52 and the third layer 56 are formed with a metal thin plate, the second layer 54 is formed with a metal thin plate in which the through-holes 55 have been formed, and, as illustrated in Fig. 3(b), an appropriate portion on the third layer 56 side is welded such that a bonding part 58 reaches the first layer 52 to form the plate-like member 50. The plate-like member 50 is curved and fixed along the stepped part 14d of a drum body 14a in each of the plurality of sections obtained by dividing the outer circumferential surface 14k of the first drum 14 in the circumferential direction.

Recess parts 13a to 13c continuous in the circumferential direction of the first drum 14 are formed on the outer circumferential surface 14k of the first drum 14 by the groove parts 53a to 53c of the first layer 52 of the plate-like member 50 (see Figs. 2(a) and 3). Bottom surfaces 13p to 13r of the recess parts 13a to 13c are formed by the second layer 54 of the plate-like member 50 (see Fig. 2(b)), and suction holes 13x (see Fig. 4) are formed in the bottom surfaces 13p to 13r of the recess parts 13a to 13c by the through-holes 55 (see Fig. 3) of the second layer 54. The spray ports 12a to 12c of the granular material supply device 12 are arranged so as to face the recess parts 13a to 13c, respectively.

The protrusion parts 14m, 14n, 14p, and 14q adjacent to the recess parts 13a to 13c are formed by the portions 52a, 52b, 52p, and 52q adjacent to the groove parts 53a to 53c of the first layer 52 of the plate-like member 50. Note that, in Fig. 2, illustration of the suction holes 13x (see Fig. 4) is omitted.

As illustrated in Figs. 1 and 2, a suction box 15 is disposed inside the first drum 14. The suction box 15 includes an internal space 15a that includes an opening 15x formed to face the plate-like member 50 and extend along the first sheet 2 wound around the first drum 14, and communicates with the opening 15x. The internal space 15a of the suction box 15 is set to a negative pressure. The first sheet 2 wound around the outer circumferential surface 14k of the first drum 14 is suctioned when the through-holes 55 of the plate-like member 50 communicate with the internal space 15a.

As illustrated in Fig. 2, the first sheet 2 covers the recess parts 13a to 13c, and is wound around the outer circumferential surface 14k of the first drum 14 so as to be supported by the protrusion parts 14m, 14n, 14p, and 14q.

Next, an operation of the apparatus for manufacturing absorbent body 10 will be described.

The apparatus for manufacturing absorbent body 10 applies adhesion to the one principal surface 2s of the first sheet 2 by the first adhesive application device 19a, and conveys the other principal surface 2t of the first sheet 2 while supporting and suctioning the other principal surface 2t of the first sheet 2 by the outer circumferential surface 14k of the first drum 14. The granular material supply device 12 sprays a granular material to the one principal surface 2s of the first sheet 2 from the spray ports 12a to 12c.

Fig. 4(a) illustrates an explanatory view for a time of spraying the granular material. As illustrated in Fig. 4(a), the spray port 12k of the granular material supply device 12 is disposed so as to face the recess parts 13a to 13c on the outer circumferential surface 14k of the first drum 14.

The first sheet 2 is curved by being suctioned by the suction holes 13x formed in the bottom surface 13k of the recess part 13 on the outer circumferential surface 14k of the first drum 14, a dent is formed in the one principal surface 2s, and the granular material is sprayed in this dent as indicated by an arrow 12x. Hence, compared to a case where the recess part 13 is not formed in the outer circumferential surface 14k of the first drum 14, that is, a case where a dent is not formed in the one principal surface 2s of the first sheet 2, scattering of the granular material to the outer side of a spray region is suppressed.

For example, the granular material sprayed from the spray port 12k of the granular material supply device 12 to the vicinity of a boundary of the recess part 13 bounces back toward the inner side of the spray region at an inclined portion of the one principal surface 2s of the first sheet 2 as indicated by an arrow 12y, and is suppressed from scattering to the outer side of the spray region. Furthermore, even when the spray amount of the granular material per unit area increases, the granular material tends to stay in the dent of the first sheet 2, so that scattering of the granular material is suppressed. Scattering of the granular material to the outer side of the spray region is suppressed as the spray port 12k of the granular material supply device 12 is placed closer to the one principal surface 2s of the first sheet 2.

A depth D to the bottom surface 13k of the recess part 13 is preferably made smaller than a thickness T of the first sheet 2. In this case, the dent at a portion of the first sheet 2 facing the recess part 13 is shallow, so that it is possible to more precisely spray the granular material to a desired position of the first sheet 2.

The guide roll 18 guides the second sheet 4 such that the one principal surface 4s of the second sheet 4 overlaps the one principal surface 2s of the first sheet 2 on which the granular material has been sprayed, thereby forming the sheet laminated body 6. The pressing roll 19 presses the sheet laminated body 6 to reliably bond the first sheet 2 and the second sheet 4.

Fig. 4(b) illustrates an explanatory view for a time of pressing the sheet laminated body 6. As illustrated in Fig. 4(b), in the sheet laminated body 6, the sprayed granular material 3 is sandwiched between the first sheet 2 and the second sheet 4, and pressed in the thickness direction by the guide roll 18 and the pressing roll 19.

The recess part 13 is formed on the outer circumferential surface 14k of the first drum 14, so that, when the guide roll 18 and the pressing roll 19 press the granular material 3, it is possible to reduce a compression amount of the portions 2b and 4b of the first sheet 2 and the second sheet 4 sandwiching the granular material 3. Consequently, even if the adhesive to be applied to the portions 2b and 4b sandwiching the granular material 3 is increased, it is possible to prevent the adhesive from oozing out, and it is easy to strengthen fixation of the granular material.

The guide roll 18 and the pressing roll 19 need to be disposed with a predetermined gap from the first drum 14. Even if the guide roll 18 and/or the pressing roll 19 hit the outer circumferential surface 14k of the first drum 14, the suction holes 13x are formed and do not hit the bottom surface 13k of the recess part 13 that is readily deformed and damaged, so that the first drum 14 is not damaged.

The second drum 21 receives the sheet laminated body 6 from the first drum 14, and conveys the sheet laminated body 6 while supporting the other principal surface 4t of the second sheet 4 of the sheet laminated body 6. The press rolls 24 and 26 press the other principal surface 2t of the first sheet 2 of the sheet laminated body 6 to compress the sheet laminated body 6 in the thickness direction.

The first press unit 31 compresses the sheet laminated body 6 in the thickness direction when the sheet laminated body 6 passes between the pair of press rolls 32 and 34. Thus, the absorber 8 is formed.

It is preferable that heaters 36a and 36b are provided inside the first press unit 31 or closer to the upstream side than the first press unit 31, and the first press unit 31 compresses the sheet laminated body 6 in the thickness direction over an entire width direction intersecting a conveyance direction of the sheet laminated body 6. In this case, it is possible to heat the sheet laminated body 6 by the heaters 36a and 36b to enhance the fluidity of the adhesive, promote diffusion of the adhesive in the entire sheet laminated body 6, and form the absorber 8 to which the granular material has been firmly fixed. Consequently, the granular material 3 does not move in the absorber 8, so that it is possible to suppress variations in the quality of the absorber 8.

In the apparatus for manufacturing absorbent body 10, a plurality of the recess parts 13a to 13c are formed on the outer circumferential surface 14k of the first drum 14 at intervals from each other, so that the absorber 8 including the channel region to which the absorbent material is not sprayed can be formed between the regions to which the absorbent material has been sprayed.

Note that the suction holes 13x can be also formed in the protrusion parts 14m, 14n, 14p, and 14q. Naturally, it is preferable that the suction holes 13x are not formed in the protrusion parts 14m, 14n, 14p, and 14q of the outer circumferential surface 14k of the first drum 14 such that the sprayed granular material is not suctioned in other than the spray region.

<Summary> As described above, the apparatus for manufacturing absorbent body 10 can suppress scattering of the granular material and oozing of the adhesive. Consequently, it is possible to reduce waste of a material, avoid stoppage of the manufacturing line, and improve production efficiency.

Note that the present invention is not limited to the above embodiment, and can be variously modified and implemented.

For example, one, two, or four or more recess parts may be formed on the outer circumferential surface of the first drum. Furthermore, a plurality of spray ports of the granular material supply device may be disposed so as to face one recess part formed on the outer circumferential surface of the first drum.

### Reference Signs List

2 first sheet
2s one principal surface
3 granular material
4 second sheet
4s one principal surface
6 sheet laminated body
10 apparatus for manufacturing absorbent body
12 granular material supply device
12a, 12b, 12c, 12k spray port
13, 13a, 13b, 13c recess part
13k, 13p to 13r bottom surface
13x suction hole
14 first drum
14k outer circumferential surface
14m, 14n, 14p, 14q protrusion part
18 guide roll
19a, 19b adhesive application device
31 first press unit (press unit)
36a, 36b heater
D depth
T thickness

## Claims

1. An apparatus for manufacturing absorbent body comprising:
a first drum that includes an outer circumferential surface on which a suction hole has been formed, and conveys a first sheet wound around the outer circumferential surface while suctioning the first sheet;
a granular material supply device that includes a spray port arranged so as to face the outer circumferential surface of the first drum, and sprays a granular material containing an absorbent material from the spray port to one principal surface of the first sheet;
a guide roll that is disposed closer to a downstream side in a rotation direction of the first drum than the spray port of the granular material supply device so as to face the outer circumferential surface of the first drum, and guides a second sheet such that one principal surface of the second sheet overlaps the one principal surface of the first sheet on which the granular material has been sprayed to form a sheet laminated body;
an adhesive application device that applies an adhesive to one or both of the one principal surface of the first sheet and the one principal surface of the second sheet before the one principal surface of the second sheet overlaps the one principal surface of the first sheet; and
a press unit that compresses the sheet laminated body in a thickness direction, wherein
in a region of the outer circumferential surface of the first drum around which the first sheet is wound, a recess part that is continuous in a circumferential direction of the first drum is formed,
the suction hole is formed in a bottom surface of the recess part, and
the spray port of the granular material supply device is disposed so as to face the recess part.

2. The apparatus for manufacturing absorbent body according to claim 1, wherein a depth to the bottom surface of the recess part of the first drum is smaller than a thickness of the first sheet.

3. The apparatus for manufacturing absorbent body according to claim 1, further comprising a heater that is inside the press unit or closer to an upstream side in the conveyance direction of the sheet laminated body than the press unit,
wherein the press unit compresses the sheet laminated body in the thickness direction over an entire width direction intersecting a conveyance direction of the sheet laminated body.

4. The apparatus for manufacturing absorbent body according to claim 1, wherein
a plurality of the recess parts are formed at intervals from each other in the region of the outer circumferential surface of the first drum, and
the granular material supply device includes a plurality of the spray ports arranged so as to face the respective recess parts.
